# EUROPÄISCHE PATENTSCHRIFT

(11) **EP 1 740 585 B1**
(45) Veröffentlichungstag und Bekanntmachung des Hinweises auf die Patenterteilung: **19.08.2009**
(21) Anmeldenummer: 05747391.0
(22) Anmeldetag: 28.04.2005
(51) Int. Cl.: C07D 471/04, A61K 31/437, A61P 25/28

(54) **ENANTIOMERENREINE HEXAHYDROPYRROLOCYCLOPENTAPYRIDIN-DERIVATE**
ENANTIOMER-PURE HEXAHYDROPYRROLOCYCLOPENTAPYRIDINE DERIVATIVES
DERIVES D'HEXAHYDROPYRROLOCYCLOPENTAPYRIDINE EXEMPTS D'ENANTIOMERES

(30) Priorität: 29.04.2004 AT 7472004; 29.04.2004 AT 7462004
(43) Veröffentlichungstag der Anmeldung: 10.01.2007
(73) Patentinhaber: Binder, Eva, 1190 Wien (AT)
(72) Erfinder: PYERIN, Michael, A-2345 Brunn am Gebirge (AT); BINDER, Dieter, verstorben (AT)
(74) Vertreter: Sonn & Partner Patentanwälte
(86) Internationale Anmeldenummer: PCT/EP2005/051921
(87) Internationale Veröffentlichungsnummer: WO 2005/105800

(56) Entgegenhaltungen:
- WO-A-20/04105766
- ZHAI, HONGBIN ET AL: "A Facile Synthesis of cis-1-Methyl-1,2,3,3a,4,8b- hexahydropyrrolo[3,2-f]pyrindine, an Annulated Nicotine Analog A Facile Synthesis of cis-1-Methyl-1,2,3,3a,4,8b- hexahydropyrrolo[3,2-f]pyrindine, an Annulated Nicotine Analog" ORGANIC LETTERS , 4(25), 4385-4386 CODEN: ORLEF7; ISSN: 1523-7060, 2002, XP002340859
- ULLRICH T ET AL: "Conformationally constrained nicotines: Polycyclic, bridged, and spiro-annulated analogues as novel ligands for the nicotinic acetylcholine receptor" EMBASE, 2002, XP002298059 & JOURNAL OF MEDICAL CHEMISTRY, Bd. 45, 2002, Seiten 4047-4054,

## Beschreibung

Nikotinderivate als mögliche Wirkstoffe bei neurodegenerativen Krankheiten, beispielsweise der Alzheimer Erkrankung, werden in der Publikation Ullrich et al. (J. Med. Chem. (2002) 45, 4047 - 4054) beschreiben.

Aufgabe der vorliegenden Erfindung ist es verbesserte Wirkstoffe zur Behandlung von Krankheiten des zentralen Reizleitungssystems bereitzustellen.

Die Erfindung betrifft neue therapeutisch wertvolle enantiomerenreine [3aα,8bα]-1,2,3,3a,4,8b-Hexahydropyrrolocyclopentapyridin-Derivate welche in die Gruppe von Verbindungen der allgemeinen Formel 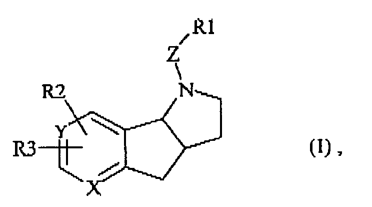 fallen, worin:
- Z: eine Einfachbindung oder CH₂,
- R1: Wasserstoff oder einen geradkettigen oder verzweigten, gegebenenfalls ungesättigten Niederalkylrest, welcher auch perfluoriert sein kann,
- R2 und R3: unabhängig voneinander Wasserstoff, einen geradkettigen oder verzweigten, gegebenenfalls ungesättigten Niederalkylrest, welcher auch perfluoriert sein kann, Niederalkoxy, Niederalkylthio oder Halogen,
- X und Y: alternierend CH oder N bedeutet,
sowie deren pharmazeutisch verwendbaren Salze.

Gegenstand der Erfindung sind neue therapeutisch wertvolle enantiomerenreine Verbindungen (-)-[3aα,8bα]-1,2,3,3a,4,8b-Hexahydro-1-methyl-pyrrolo[2',3':3,4]-cyclopenta[1,2-b]pyridin, (-)-[3aR-(3aα,8bα)]-1,2,3,3a,4,8b-Hexahydro-pyrrolo[3',2':4,5]-cyclopenta[1,2-c]pyridin oder (-)-[3aR-(3aα,8bα)]-1,2,3,3a,4,8b-Hexahydro-1-methyl-pyrrolo[3',2':4,5]-cyclopenta[1,2-c]pyridin, sowie deren pharmazeutisch verwendbaren Salze.

Die enantiomerenreinen Verbindungen bzw. Präparationen gemäß der vorliegenden Erfindung weisen gegenüber den Enantiomeren-Gemischen bzw. Racematen dieser Verbindungen überraschen positive Eigenschaften, vor allem im Hinblick auf ihre biologische Aktivität, insbesondere im ZNS-Bereich, auf. Es zeigte sich, dass die jeweiligen Enantiomeren einen starken Wirkungsunterschied aufweisen, wie auch in Beispiel 9 demonstriert wird.

Weiters bezieht sich die vorliegende Erfindung auf ein Verfahren zur Herstellung dieser Verbindungen, welches dadurch gekennzeichnet ist, dass man eine Verbindung der allgemeinen Formel 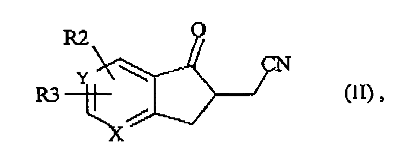 worin R2, R3 Wasserstoff sind und X und Y alternierend CH oder N bedeuten, reduktiv in die Verbindung der allgemeinen Formel (I) mit Z = Einfachbindung und R1 = Wasserstoff überführt, diese gegebenenfalls mit enantiomerenreinem 1-Phenylethylisocyanat zur Verbindung der allgemeinen Formel 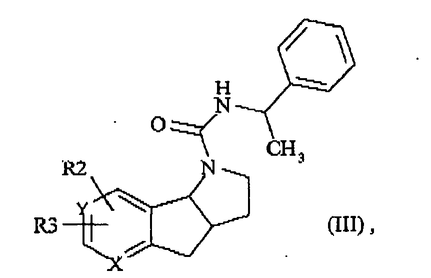 umsetzt, aus dem so erhaltenen Diastereomerengemisch das schwerer lösliche Diastereomere durch Kristallisation gewinnt, die so erhaltene diastereomerenreine Verbindung der allgemeinen Formel (III) unter geeigneten Bedingungen zur enantiomerenreinen Verbindung der allgemeinen Formel (I) mit Z = Einfachbindung und R1 = Wasserstoff spaltet, diese gegebenenfalls unter alkylierenden Bedingungen zu Verbindungen der allgemeinen Formel (I) mit Z = CH₂ umsetzt, und gegebenenfalls die Verbindung der allgemeinen Formel (I) in ihre pharmazeutisch verwendbaren Salze überführt.

Der oben verwendete Ausdruck "Niederalkyl" bedeutet einen geradkettigen oder verzweigten Alkylrest mit 1-4 Kohlenstoffatomen, beispielsweise Methyl, Ethyl, n- und i-Propyl, n-, i- und t-Butyl.
Der oben verwendete Ausdruck "Niederalkoxy" bedeutet einen geradkettigen oder verzweigten Alkoxyrest mit 1-4 Kohlenstoffatomen, beispielsweise Methoxy, Ethoxy, n- und i-Propoxy, n-, i- und t-Butoxy.

Der oben verwendete Ausdruck "Niederalkylthio" bedeutet einen geradkettigen oder verzweigten Alkylthiorest mit 1-4 Kohlenstoffatomen, beispielsweise Methylthio, Ethylthio, n- und i-Pxopylthio, n-, i- und t-Butylthio.
Der oben verwendete Ausdruck "Halogen" bedeutet Fluor, Chlor, Brom oder Iod.

Die erfindungsgemäßen Umsetzungen werden am besten so durchgeführt, dass man die Verbindung der allgemeinen Formel (U) in einem polaren Lösungsmittel, wie z.B. Essigsäureethylester, Dioxan, Ethanol oder Methanol löst, mit 1-5 Äquivalenten eines geeigneten Katalysators, wie z.B. W2-Raney-Nickel oder Raney Cobalt u.ä. versetzt und bei 40 bis 70°C bis zur stöchiometrischen Wasserstoffaufnahme hydriert.
Die so erhaltene racemische Verbindung der allgemeinen Formel (I) mit Z = Einfachbindung und R1 = Wasserstoff kann zur Enantiomerentrennung in einem inerten Lösungsmittel, wie z.B. Tetrahydrofuran, Dioxan oder Aceton mit 1 Äquivalent (+) oder (-) 1-Phenylethylisocyanat zu einer Verbindung der allgemeinen Formel (III) umgesetzt und aus dem so erhaltenen Diastereomerengemisch das schwerer lösliche Diastereomere durch Kristallisation gewonnen werden. Die so erhaltene diastereomerenreine Verbindung der allgemeinen Formel (III) wird zur Spaltung in einem hochsiedendem Alkohol, wie z.B. Propanol, Butanol, Pentanol, Glycol usw. oder deren wäßrigen Gemischen gelöst und mit 5-20 Äqivalenten einer Base, wie Natriumpropanolat, -butanolat, -pentanolat oder Natriumhydroxid für 1-24 Stunden zum Sieden erhitzt.
Die so erhaltene enantiomerenreine Verbindung der allgemeinen Formel (I) mit **Z** = Einfachbindung und **R1** = Wasserstoff, wird gegebenenfalls zur Alkylierung in einem inerten Lösungsmittel, wie z.B. Tetrahydrofuran, Dioxan, Acetonitril oder Dimethylformamid usw. gelöst, mit 1-20 Äquivalente der Verbindung der Formel

R1-CHO (IV),

worin **R1** obige Bedeutung hat, und 1.5-4 Äquivalenten eines Reduktionsmittels, wie z.B. Natriumcyanoborhydrid o.ä. versetzt und bei -20°C bis 100°C zwischen 1 und 24 Stunden umgesetzt.

Die bei dieser Umsetzung erhaltenen Verbindungen der allgemeinen Formel (I) sind basische Verbindungen und können auf übliche Weise mit anorganischen oder organischen Säuren in ihre pharmazeutisch verträglichen Salze überführt werden.
Die Salzbildung kann beispielsweise durchgeführt werden, indem man die Verbindungen der Formel (I) in einem geeigneten Lösungsmittel, z.B. Wasser, einem niederen aliphatischen Alkohol, THF, Dioxan, Benzol, Diethylether, DMF oder DMSO löst, eine äquivalente Menge der gewünschten Säure zusetzt, für eine gute Durchmischung sorgt und nach beendeter Salzbildung das Lösungsmittel im Vakuum abzieht. Gegebenenfalls können die Salze nach der Isolierung umkristallisiert werden.

Pharmazeutisch verwendbare Salze sind jene starker anorganischer Säuren, wie z.B. Salzsäure, Bromwasserstoffsäure, Schwefelsäure usw., aber auch solche organischer Säuren wie z.B. Fumarsäure, Zitronensäure, Sebacinsäure usw.

Die Verbindungen der allgemeinen Formel (II) mit **X** = N und **Y** = CH, können beispielsweise nach **Schema 1** erhalten werden (analog E. Schröder M. Lehrmann und I. Böttcher, Eur. J. Med. Chem. 1979, **14**(4), 309-15, J.Hurst und D.G. Wibberly, J. Chem. Soc. 1962, 119). Demnach wird der gegebenfalls mit **R2** und **R3** substituierte 2-Methyl-3-nicotinsäureester der allgemeinen Formel (V), worin **R4** einen Niederalkylrest bedeutet, mit 1.05 Äq NBS in siedendem Tetrachlorkohlenstoff radikalisch bromiert und das Rohprodukt mit 1.0 Äq Natriummalonat in N,N-Dimethylformamid substituiert. Der erhaltene Triester der allgemeinen Formel (VI), worin **R5** einen Niederalkylrest bedeutet, wird unter Einwirkung von 1.05 Äq Natriumhydrid in siedendem Tetrahydrofuran einer Dieckmannschen Esterkondensation unterworfen und durch quenchen mit wäßrige Ammoniumchloridlösung zum β-Ketoester (VII) monoverseift und decarboxyliert. Nach Deprotonierung mit Natriumhydrid und Alkylierung mit Jodacetonitril in N,N-Dimethylformamid wird durch Erhitzen in 2n Salzsäure zur Verbindung der allgemeinen Formel (II) verseift und decarboxyliert.

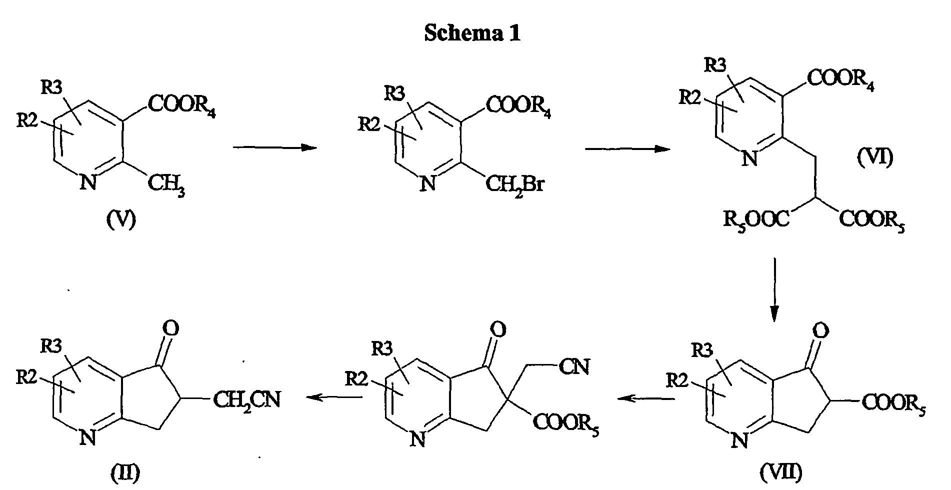

Die Verbindungen der allgemeinen Formel (II) mit **X** = CH und **Y** = N, können beispielsweise nach **Schema 2** ausgehend von dem, gegebenfalls mit **R2** und **R3** substituierten Nicotinsäureester der allgemeinen Formel (VIII), worin **R4** einen Niederalkylrest bedeutet, erhalten werden. Dieser wird mit 1.0 Äq Chlorameisensäureester aktiviert und mit dem Zink-Kupfer-Organyl eines 3-Jodpropionsäureester in Tetrahydrofuran zum 1,4-Dihydropyridin umgesetzt, welches mit Schwefel in siedendem Xylol zum Pyridin der allgemeinen Fromel (IX), worin **R5** einen Niederalkylrest bedeutet, oxidiert wird (analog M. J. Shiao, W. L. Chia, C. J. Peng und C. C. Shen, J. Org. Chem. 1933, 58, 3162-4). Dieses wird unter Einwirkung von 1.3 Äq Natriummethanolat in siedendem Methanol in einer Dieckmannschen Esterkondensation zum β-Ketoester (X) zyklisiert (analog D. Binder, Monatshefte für Chemie 1974, 105, 196-202). Nach Deprotonierung mit Natriumhydrid und Alkylierung mit Jodacetonitril in N,N-Dimethylformamid wird durch Erhitzen in 2n Salzsäure zur Verbindung der allgemeinen Formel (II) verseift und decarboxyliert.

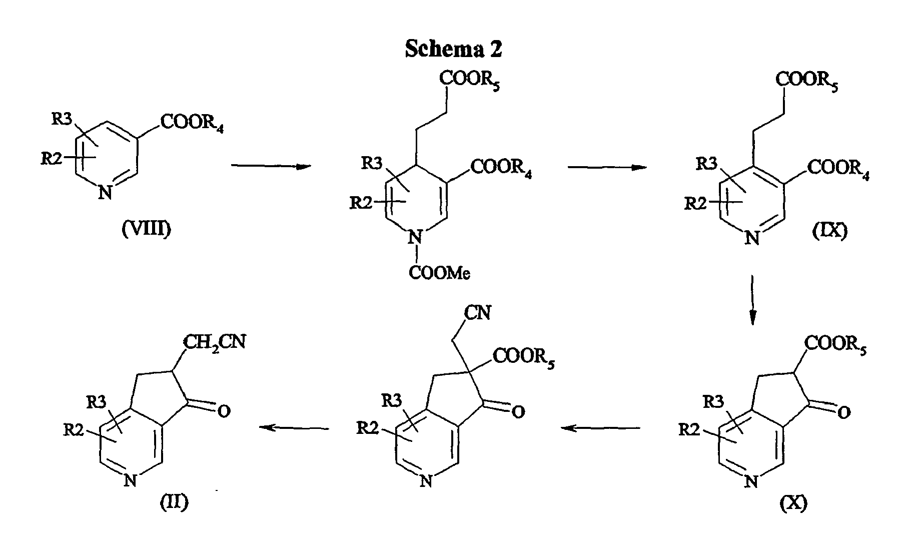

Die Verbindungen der allgemeinen Formel (IV), (V) und (V) sind litsraturbekannt oder können analog dazu nach üblichen und dem Fachmann geläufigen Methoden hergestellt werden.

Die erfindungsgemäßen Verbindungen der allgemeinen Formel (I) und deren Salze sind Agonisten von zentralen Nicotin-Rezeptor-Subtypen und sind daher zur Behandlung von Erkrankungen des zentralen Reizleitungssystemes wie z.B. altersbedingte Demenz, Alzheimer-Krankheit, Parkinson-Krankheit, Tourett's-Syndrom, Dyskinesie, Angstzustände, Depression, Panik, Psychosen, Bulemie, Anorexie und als Analgetika, Nozizieptiva, Neuroprotektiva, zur Verbesserung von Wahrnehmung und Aufmerksamkeit sowie in der Rauch-Ersatztherapie usw. besondes gut geeignet.

Aufgrund dieser pharmakologischen Eigenschaften können die neuen Verbindungen allein oder in Mischung mit anderen Wirksubstanzen in Form üblicher galenischer Zubereitungen als Heilmittel zur Behandlung von Erkrankungen, die durch Aktivierung des Systems der zentralen Nicotinrezeptorsubtypen geheilt oder gelindert werden, oder als Analgetika, Nozizieptiva, Neuroprotektiva, zur Verbesserung von Wahrnehmung und Aufmerksamkeit sowie in der Rauch-Ersatztherapie Verwendung finden.

Die Erfindung bezieht sich weiterhin auf Heilmittel, die z.B. in Form pharmazeutischer Präparate Verwendung finden, welche die erfindungsgemäßen Verbindungen der allgemeinen Formel (I) und ihre Salze in Mischung mit einem für die orale, enterale, parenterale und topicale Applikation geeigneten, pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline oder dergleichen enthalten.

Die pharmazeutischen Präparate können in fester Form z.B. als Tabletten, Filmtabletten, Dragees, Suppositorien, Kapseln, Mikrokapseln oder in flüssiger Form z.B. als Lösungen, Injektionslösungen, Suspensionen oder Emulsionen oder in Zusammensetzungen mit verzögerter Freigabe des Wirkstoffes vorliegen.
Gegebenenfalls sind sie sterilisiert und/oder enthalten Hilfsstoffe, wie Konservierungs-, Stabilisierungs- oder Emulgiermittel, Salze zur Veränderung des osmotischen Druckes oder Puffer.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen zusammen mit den oben angegebenen Hilfs- und/oder Trägerstoffen zu Kombinationspräparaten formuliert werden.

Die neuen Verbindungen können in den erfindungsgemäßen pharmazeutischen Zusammensetzungen in einem Anteil von etwa 1-200 mg pro Tablette vorhanden sein, wobei der Rest ein pharmazeutisch annehmbarer Füllstoff ist.

Eine geeignete Dosis zur Verabreichung der neuen Verbindungen beträgt etwa 1-200 mg/kg pro Tag, jedoch kommen, je nach dem Zustand des zu behandelnden Patienten, auch andere Dosen in Frage. Die neuen Verbindungen können in mehreren Dosen und auf oralem Weg verabreicht werden.

Die folgenden Beispiele erläutern die Erfindung näher, ohne dass diese darauf beschränkt sein soll:

### Vergleichsbeispiel 1

### (+)- [3aα, 8bα]- 1, 2, 3, 3a, 4, 8b- Hexahydropyrrolo [2', 3': 3, 4] cyclopenta [1, 2-b] pyridin- Dihydrochlorid

6.68 g (278 mmol) Natriumhydrid werden bei 0 °C mit 280 ml absolutem Pentanol versetzt und 30 Minuten bei Raumtemperatur gerührt. 5.34 g (17.4 mmol) (-)-[1(S),3aα,8bα]-1,2,3,3a,4,8b-Hexahydro-N-(1-phenylethyl)-pyrrblo[2',3':3,4]cyclopenta[1,2-b]pyridin-1-carbonsäureamid werden in fester Form unter N₂-Gegenstromspülung auf einmal zugegeben und das Reaktionsgemisch sofort zwei Stunden zum Sieden erhitzt.
Das Lösungsmittel wird bei 60 °C/ 0.1 mbar abgezogen und der Rückstand rasch über 400 g Kieselgel mit Methanol: Ammoniak= 100: 2 filtriert. Das Lösungsmittel wird abgezogen und das Rohprodukt säulenchromatographisch durch Gradientenelution gereinigt (400 g Kieselgel, Dichlormethan: Methanol= 1: 1 -> Methanol -> Methanol: Ammoniak= 100: 2).
Das erhaltene Produkt wird in 20 ml Dichlormethan aufgenommen, über Natriumsulfat/Aktivkohle getrocknet, filtriert und das Lösungsmittel abdestilliert.
***Ausbeute:*** 2.23 g hellbeige Kristalle (82 % d. Th.)
***DC:*** Methanol: Ammoniak= 100: 2; R_{f} = 0.5

Das Produkt wird mit alkoholischer Salzsäure in sein Dihydrochlorid überführt, unter Ethanol kristallisiert, abfiltriert und mit Aceton digeriert. Die erhaltenen farblosen Kristalle sind stark hygroskopisch.

### [α]_{D}²⁰: + 14.4 ± 1.1 ° (c = 0.22/ Methanol)

| ***Mikroelemtaranlyse:*** | RW5 | *C(%)* | *H(%)* | *N(%)* |
|---|---|---|---|---|
| *Summenformel:* | *berechnet:* | 51.52 | 6.05 | 12.02 |
| C₁₀H₁₄Cl₂N₂ | *gefunden:* | 51.52 | 6.10 | 11.81 |

### ¹H- NMR (D₂O):

δ (ppm) = 8.73 (d,1H,Pcp-H6); 8.69 (d,1H,Pcp-H8); 7.94 (dd,1H,Pcp-H7); 5.54 (d,1H,Pcp-H8b); 3.80-3.20 (m,5H,Pcp-H2,3a,4A,4B); 2.58-2.36 (m,1H,Pcp-H3A); 2.09-1.90 (m,1H,Pcp-H3B)

### ¹³C NMR (D₂O):

δ (ppm) = 156.2 (s,Pcp-C4a); 142.5 (d,Pcp-C6); 139.7 (d,Pcp-C8); 133.3 (s,Pcp-C8a); 123.1 (d,Pcp-C7); 62.2 (d,Pcp-C8b); 43.5 (t,Pcp-C2); 36.6 (d,Pcp-C3a); 33.1 (t,Pcp-C4); 28.3 (t,Pcp-C3)

### Vergleichsbeispiel 2

### (-)- [3aα, 8bα]- 1, 2, 3, 3a, 4, 8b- Hexahydropyrrolo [2', 3': 3, 4] cyclopenta [1, 2-b] pyridin- Dihydrochlorid

aus 9.10 g (30.0 mMol) (+)-[1(R),3aα,8bα]-1,2,3,3a,4,8b-Hexahydro-N-(1-phenylethyl)-pyrrolo[2',3':3,4]cyclo-penta[1,2-b]pyridin-1-carbonsäureamid analog zu Vergleichsbeispiel 1 (72% d.Th., farblose Kristalle)

### [α]_{D}²⁰: - 13.9 ± 1.0 ° (c = 0.24/ Methanol)

| ***Mikroelemtaranlyse:*** | RW7 | *C(%)* | *H(%)* | *N(%)* |
|---|---|---|---|---|
| *Summenformel:* | *berechnet:* | 51.16 | 6.09 | 11.93 |
| C₁₀H₁₄Cl₂N₂ * 0.09 H₂O | *gefunden:* | 51.22 | 6.24 | 11.80 |

### ¹H NMR (D₂O):

δ (ppm) = 8.73 (d,1H,Pcp-H6); 8.69 (d,1H,Pcp-H8); 7.94 (dd,1H,Pcp-H7); 5.54 (d,1H,Pcp-H8b); 3.80-3.20 (m,5H,Pcp-H2,3a,4A,4B); 2.58-2.36 (m,1H,Pcp-H3A); 2.09-1.90 (m,1H,Pcp-H3B)

### ¹³C- NMR (D₂O):

δ (ppm) = 156.2 (s,Pcp-C4a); 142.5 (d,Pcp-C6); 139.7 (d,Pcp-C8); 133.3 (s,Pcp-C8a); 123.1 (d,Pcp-C7); 62.2 (d,Pcp-C8b); 43.5 (t,Pcp-C2); 36.6 (d,Pcp-C3a); 33.1 (t,Pcp-C4); 28.3 (t,Pcp-C3)

### Das Ausgangsmaterial kann wie folgt hergestellt werden:

### 2-[2,2-Bis-(methoxycarbonyl)]-ethyl-3-pyridincarbonsäuremethylester

219.0 g (1.45 mol) 2-Methyl-3-pyridincarbonsäuremethylester in 3.5 1 absolutem Tetrachlormethan werden mit 271.0 g (1,52 mol) N-Bromsuccinimid und 13.0 g Dibenzoylperoxid über Nacht zum Sieden erhitzt.
Der Feststoff wird abfiltriert, das Lösungsmittel abgezogen, der Rückstand in 250 ml absolutem Dimethylformamid gelöst und bei 10 °C zu einer Suspension von 223.3 g (1.45 mol) Natriumdimethylmalonat in 1.2 1 absolutem Dimethylformamid zugetropft und 18 Stunden bei Raumtemperatur gerührt.
Das Lösungsmittel wird im Hochvakuum abgezogen, der Rückstand zwischen 2.5 1 Wasser und
1.5 1 Diethylether verteilt und die wäßrige Phase viermal mit je 800 ml Diethylether extrahiert. Die vereinigten organischen Phasen werden mit 200 ml Wasser gewaschen, über Natriumsulfat/ Aktivkohle getrocknet, filtriert, das Lösungsmittel abgezogen und der Rückstand mit 200 ml Diethylether digeriert.
***Ausbeute:*** 138.5 g farblose Kristalle (34 % d.Th.)
***DC:*** Essigsäureethylester; R_{f} = 0.6
***Fp.:*** 59-62 °C (Diethylether, dig.)

| ***Mikroelemtaranlyse:*** | HK29 | *C(%)* | *H(%)* | *N(%)* |
|---|---|---|---|---|
| *Summenformel:* | *berechnet:* | 55,51 | 5,38 | 4,98 |
| C₁₀H₁₄Cl₂N₂ | *gefunden:* | 55,71 | 5,26 | 4,92 |

### ¹H NMR (CDCl₃):

δ (ppm) = 8.55 (dd,1H,Py-H6); 8.18 (dd,1H,Py-H4); 7.20 (dd,1H,Py-H5); 4.22 (t,1H,CH); 3.90 (s,3H,OCH₃); 3.80 (d,2H,CH₂); 3.70 (s,6H,OCH₃)

### ¹³C- NMR (CDCl₃):

δ (ppm) = 169.7 (s,2C,COOCH₃); 166.2 (s,COOCH₃); 158.4 (s,Py-C2); 151.3 (d,Py-C6); 138.3 (d,Py-C4); 125.0 (s,Py-C3); 121.2 (d,Py-C5); 52.2 (q,2C,OCH₃); 52.1 (q,OCH₃); 49.6 (d,CH); 34.9 (t,CH₂)

### 6,7-Dihydro-5-oxo-5H-1-pyrindin-6-carbonsäuremethylester

Zu einer Suspension von 8.40 g (350 mmol) Natriumhydrid in 800 ml siedendem absolutem Tetrahydrofuran werden 93.5 g (332 mmol) 2-[2,2-Bis-(methoxycarbonyl)]-ethyl-3-pyridincarbonsäuremethylester in 600 ml absolutem heißem Tetrahydrofuran, langsam zugegeben. Die Reaktionslösung wird bis zum Ende der Gasentwicklung unter Rückfluß erhitzt, wobei das Produkt ausfällt.
Die abgekühlte Suspension wird auf 1.5 1 gesättigte Ammoniumchloridlösung gegossen, 30 Minuten gerührt und der Niederschlag abfiltriert. Der Feststoff wird dreimal mit 250 ml Wasser, einmal mit 250 ml Methanol digeriert und über Phosphorpentoxid bei 70 °C/ 20 mbar getrocknet.
***Ausbeute:*** 55.4 g farblose Kristalle (87 % d.Th.)
***DC:*** Essigsäureethylester; R_{f} = 0.4
***Fp.:*** 92-96 °C (Essigsäureethylester)

| ***Mikroelemtaranlyse:*** | | *C(%)* | *H(%)* | *N(%)* |
|---|---|---|---|---|
| *Summenformel:* | *berechnet:* | 62.82 | 4.74 | 7.32 |
| C₁₀H₉NO₃ | *gefunden:* | 63.02 | 4.79 | 7.32 |

### ¹H- NMR (CDCl₃):

*Ketoform:* δ (ppm) = 8.82 (dd,1H,Pn-H2); 8.02 (dd,1H,Pn-H4); 7.35 (dd,1H,Pn-H3); 3.79 (dd,1H,Pn-H6); 3.78 (s,3H,OCH₃); 3.72-3.40 (m,2H,Pn-H7A,B)

*Eno*/*form:* δ (ppm) = 8.58 (dd,1H,Pn-H2); 7.87 (dd,1H,Pn-H4); 7.27 (dd,1H,Pn-H3); 3.86 (s,3H,OCH₃); 3.61 (s,2H,Pn-H7)

### ¹³C NMR (CDCl₃):

*Ketoform:* δ (ppm) = 197.6 (s,Pn-C5); 172.7 (s,COOCH₃); 168.8 (s,Pn-C7a); 156.3 (d,Pn-C2);
132.7 (d,Pn-C4); 128.5 (s,Pn-C4a); 122.8 (d,Pn-C3); 52.8 (d,Pn-C6); 52.7 (q,OCH₃); 32.9 (t,Pn-C7)

*Enolform:* δ (ppm) = 169.3 (s,COOCH₃); 167.1 (s,Pn-C5)*; 163.5 (s,Pn-C7a)*; 149.8 (d,Pn-C2); 131.9 (s,Pn-C4a)*; 128.1 (d,Pn-C4); 121.7 (d,Pn-C3); 102.0 (s,Pn-C6); 51.3 (q,OCH₃); 34.8 (t,Pn-C7)

### 6-(Cyanomethyl)-6,7-dihydro-5-oxo-5H-1-pyrindin-6-carbonsäuremethylester

Zu einer Suspension von 7.66 g (319 mmol) Natriumhydrid in 400 ml absolutem Dimethylformamid werden 55.4 g (290 mmol) 6,7-Dihydro-5-oxo-5H-1-pyrindin-6-carbonsäure-methylester bei 5 °C zugegeben und bis zum Ende der Gasentwicklung gerührt. Nach der Zugabe von 55.7 g (333 mmol) Jodacetonitril in 200 ml absolutem Dimethylformamid rührt man zuerst 30 Minuten bei 5 °C und anschließend 18 Stunden bei Raumtemperatur.
Man stellt mit Eisessig auf pH 5, zieht Dimethylformamid am Feinvakuum ab, nimmt den Rückstand in 900 ml Wasser auf, extrahiert zweimal mit je 600 ml und dreimal mit je 300 ml Dichlormethan. Die organische Phase wird über Natriumsulfat/ Aktivkohle getrocknet, filtriert und das Lösungsmittel abdestilliert. Das Rohprodukt wird nach Startfleckfiltration (1.2 kg Kieselgel KG60, Essigsäureethylester) aus 200 ml Essigsäureethylester umkristallisiert.
***Ausbeute:*** 35.6 g farblose Kristalle (53 % d.Th.)
***DC:*** Essigsäureethylester; R_{f} = 0.6
***Fp.:*** 99-101 °C (Essigsäureethylester)

| ***Mikroelemtaranlyse:*** | FG1 | *C(%)* | *H(%)* | *N(%)* |
|---|---|---|---|---|
| *Summenformel:* | *berechnet:* | 62.61 | 4.38 | 12.17 |
| C₁₂H₁₀N₂O₃ | *gefunden:* | 62.45 | 4.27 | 12.16 |

### ¹H- NMR (CDCl₃):

δ (ppm) = 8.90 (dd,1H,Pn-H2); 8.09 (dd,1H,Pn-H4); 7.44 (dd,1H,Pn-H3); 3.87 (d,1H,Pn-H7A); 3.71 (s,3H,OCH₃); 3.42 (d,1H,Pn-H7B); 3.20 (d,1H, CHAHB-CN); 2.98 (d,1H,CHAHB-CN)

### ¹³C- NMR (CDCl₃):

δ (ppm) = 197.4 (s,Pn-C5); 171.6 (s,COOCH₃); 168.6 (s,Pn-C7a); 157.1 (d,Pn-C2); 133.4 (d,Pn-C4); 127.5 (s,Pn-C4a); 123.4 (d,Pn-C3); 116.0 (s,CN); 56.6 (s,Pn-C6); 53.6 (q,OCH₃); 39.4 (t,Pn-C7); 22.1 (t,CH₂CN)

### 6,7-Dihydro-5-oxo-5H-1-pyrindin-6-carbonsäurenitril

35.6 g (155 mmol) 6-(Cyanomethyl)-6,7-dihydro-5-oxo-1-pyrindin-6-carbonsäuremethylester werden in 400 ml 2n Salzsäure 90 Minuten zum Sieden erhitzt.
Die Reaktionslösung wird mit festem Natriumcarbonat auf pH= 9 gestellt, zweimal mit je 200 ml und dreimal mit je 100 ml Dichlormethan extrahiert. Die organische Phase wird über Natriumsulfat/ Aktivkohle getrocknet, filtriert und das Lösungsmittel abdestilliert.
***Ausbeute:*** 24.0 g farblose Kristalle (90 % d.Th.)
***DC:*** Essigsäureethylester; R_{f} = 0.5
***Fp.:*** 95-97 °C (Dichlormethan)

| ***Mikroelemtaranlyse:*** | FG2 | *C(%)* | *H(%)* | *N(%)* |
|---|---|---|---|---|
| *Summenformel:* | *berechnet:* | 69.76 | 4.68 | 16.27 |
| C₁₀H₈N₂O | *gefunden:* | 69.54 | 4.76 | 16.20 |

### ¹H- NMR (CDCl₃):

δ (ppm) = 8.87 (dd,1H,Pn-H2); 8.06 (dd,1H,Pn-H4); 7.37 (dd,1H,Pn-H3); 3.66 (dd,1H,Pn-H7A); 3.22- 2.92 (m,3H,Pn-H7B,Pn-H6,CHAHB-CN); 2.73 (dd,1H,CHAHB-CN)

### ¹³C- NMR (CDCl₃):

δ (ppm) = 202.2 (s,Pn-C5); 171.7 (s,Pn-C7a); 156.5 (d,Pn-C2); 132.4 (d,Pn-C4); 128.9 (s, Pn-C4a); 123.0 (d,Pn-C3); 117.2 (s,CN); 42.7 (d,Pn-C6); 34.5 (t,Pn-C7); 18.3 (t,CH₂CN)

### (±)-[3aα, 8bα]- 1, 2, 3, 3a, 4, 8b- Hexahydropyrrolo [2', 3': 3, 4] cyclopenta [1, 2-b] pyridin

4.0 g (23.23 mmol) 6, 7- Dihydrö- 5- oxo- 5H- 1- pyrindin- 6- essigsäurenitril werden in 80 ml absolutem Methanol gelöst, mit Aktivkohle gerührt und filtriert. Die Lösung wird mit 16 g Raney- Cobalt- Katalysator versetzt und in einer Parr-Apparatur bei 50 °C und 90 psi Wasserstoffdruck bis zum Ende der theoretischen Wasserstoffaufnahme hydriert.
Der Katalysator wird über Hyflo abfiltriert, das Lösungsmittel des Filtrats abdestilliert und der Rückstand durch Startfleckfiltration über 400 g Kieselgel mit Methanol: Ammoniak= 100: 2 gereinigt. Das Lösungsmittel wird abdestilliert, der Rückstand in Dichlormethan aufgenommen, über Natriumsulfat/ Aktivkohle getrocknet, filtriert und das Lösungsmittel abgezogen.
***Ausbeute:*** 2.61 g farbloses Öl (70 % d.Th.)
***DC*:** Methanol: Ammoniak= 100: 2; R_{f} = 0.5

### ¹H- NMR (CDCl₃):

δ (ppm) = 8.42 (dd,1H,Pcp-H6); 7.62 (dd,1H,Pcp-H8); 7.09 (dd,1H,Pcp-H7); 4.74 (d,1H,Pcp-H8b); 3.30 (dd,1H,Pcp-H4A); 3.12- 2.61 (m,4H,Pcp-H4B,2,3a); 2.15- 1.94 (m,1H,Pcp-H3A); 1.68- 1.49 (m,1H,Pcp-H3B)

### ¹³C- NMR (CDCl₃):

δ (ppm) = 164.0 (s,Pcp-C4a); 149.3 (d,Pcp-C6); 137.4 (s,Pcp-C8a); 133.1 (d,Pcp-C8); 121.7 (d,Pcp-C7); 66.5 (d,Pcp-C8b); 46.6 (t,Pcp-C2); 40.2 (d,Pcp-C3a); 39.3 (t,Pcp-C4); 35.7 (t,Pcp-C3)

### (-)-[1(S),3aα,8bα]-1,2,3,3a,4,8b-Hexahydro-N-(1-phenylethyl)-pyrrolo[2',3':3,4]-cyclopenta[1,2-b]pyridin-1-carbonsäureamid

2.50 g (15.6 mmol) (±)-[3aα,8bα]-1,2,3,3a,4,8b-Hexahydropyrrolo[2',3':3,4]cyclopenta[1,2-b]pyridin in 30 ml absolutem Aceton werden mit 2.30 g (15.6 mmol) (S)-(-)-1- Phenylethylisocyanat in 25 ml absolutem Aceton versetzt, 60 Minuten bei Raumtemperatur gerührt und zur vollständigen Kristallisation über Nacht in den Eiskasten gestellt.
Die Kristalle werden abfiltriert und mit kaltem, absoluten Aceton digeriert.
***Ausbeute:*** 2.04 g farblose Kristalle (85% d. Th.)
***DC:*** Dichlormethan: Methanol= 95:5; R_{f} = 0.5
***Fp.:*** 187- 189 °C (Aceton)
***[α]_{D}²⁰:*** - 229.5 ± 0.5 ° (c = 1.00/ Dichlormethan)

| ***Mikroelemtaranlyse:*** | GD28 | *C(%)* | *H(%)* | *N(%)* |
|---|---|---|---|---|
| *Summenformel:* | *berechnet:* | 74.24 | 6.89 | 13.67 |
| C₁₉H₂₁N₃O | *gefunden:* | 74.24 | 7.10 | 13.53 |

### ¹H- NMR (CDCl₃):

δ (ppm) = 8.39 (d,1H,Pcp-H6); 8.03 (d,1H,Pcp-H8); 7.34- 7.21 (m,5H,Bz-H2-4); 7.06 (dd,1H,Pcp-H7); 5.36 (d,1H,Pcp-H8b); 5.05 (m,1H,CH); 4.63 (d,1H;NH); 3.36-3.29 (m,2H,Pcp-H2); 3.18 (dd,1H,Pcp-H4A); 3.10- 2.90 (m,1H,Pcp-H3a); 2.83 (dd,1H,Pcp-H4B); 2.29- 2.14 (m,1H,Pcp-H3A); 1.79- 1.60 (m,1H,Pcp-H3B); 1.48 (d,3H,CH₃)

### ¹³C- NMR (CDCl₃):

δ (ppm) = 161.9 (s,Pcp-C4a); 156.3 (s,CO); 149.0 (d,Pcp-C6); 144.4 (s,Bz-Cl); 137.3 (s,Pcp-C8a); 135.6 (d,Pcp-C8); 128.4 (d,2C,Bz-C3); 126.9 (d,Bz-C4); 125.9 (d,2C,Bz-C2); 121.9 (d,Pcp-C7); 64.5 (d,Pcp-C8b); 49.7 (d,CH); 45.7 (t,Pcp-C2); 39.0 (d,Pcp-C3a); 37.8 (t,Pcp-C4); 31.3 (t,Pcp-C3); 22.5 (q,CH₃)

### (+)-[1(R),3aα,8bα]-1,2,3,3a,4,8b-Hexahydro-N-(1-phenylethyl)-pyrrolo[2',3':3,4]-cyclopenta[1,2-b]pyridin-1-carbonsäureamid

aus 5.12 g (31.9 mmol) (±)-[3aα,8bα]-1,2,3,3a,4,8b-Hexahydropyrrolo[2',3':3,4]cyclopenta-[1,2-b]pyridin und 4.47 g (30.3 mol) (R)-(+)-1-Phenylethylisocyanat analog zu (-)-[1(S),3aα,8bα]-1,2,3,3a,4,8b-Hexahydro-N-(1-phenylethyl)-pyrrolo[2',3':3,4]-cyclopenta[1,2-b]pyridin-1-carbonsäureamid
***Ausbeute:*** 4.19 g farblose Kirstalle (85 % d. Th.)
***DC:*** Dichlormethan: Methanol= 95: 5; R_{f} = 0.5
***Fp.:*** 184- 186 °C (Aceton)
***[*α*]_{D}²⁰:*** + 230.4 ± 0.5 ° (c = 0.19/ Dichlormethan)

| ***Mikroelemtaranlyse:*** | RW1 | *C(%)* | *H(%)* | *N(%)* |
|---|---|---|---|---|
| *Summenformel:* | *berechnet:* | 74.24 | 6.89 | 13.67 |
| G₁₉H₂₁N₃O | *gefunden:* | 74.26 | 7.04 | 13.56 |

### ¹H- NMR (CDCl₃):

δ (ppm) = 8.39 (d,1H,Pcp-H6); 8.03 (d,1H,Pcp-H8); 7.34- 7.21 (m,5H,Bz-H2-4); 7.06 (dd,1H,Pcp-H7); 5.36 (d,1H,Pcp-H8b); 5.05 (m,1H,CH); 4.63 (d,1H;NH); 3.36-3.29 (m,2H,Pcp-H2); 3.18 (dd,1H,Pcp-H4A); 3.10- 2.90 (m,1H,Pcp-H3a); 2.83 (dd,1H,Pcp-H4B); 2.29- 2.14 (m,1H,Pcp-H3A); 1.79- 1.60 (m,1H,Pcp-H3B); 1.48 (d,3H,CH₃)

### ¹³C- NMR (CDCl₃):

δ (ppm) = 161.9 (s,Pcp-C4a); 156.3 (s,CO); 149.0 (d,Pcp-C6); 144.4 (s,Bz-C1); 137.3 (s,Pcp-C8a); 135.6 (d,Pcp-C8); 128.4 (d,2C,Bz-C3); 126.9 (d,Bz-C4); 125.9 (d,2C,Bz-C2); 121.9 (d,Pcp-C7); 64.5 (d,Pcp-C8b); 49.7 (d,CH); 45.7 (t,Pcp-C2); 39.0 (d,Pcp-C3a); 37.8 (t,Pcp-C4); 31.3 (t,Pcp-C3); 22.5 (q,CH₃)

### Beispiel 1

### (-)- [3aα, 8bα]-1, 2, 3, 3a, 4, 8b- Hexahydro-1-methyl-pyrrolo [2', 3': 3, 4] cyclopenta [1, 2- b] pyridin- Dihydrochlorid

1.15 g (7.17 mmol) [3aα,8bα]-1,2,3,3a,4,8b-Hexahydropyrrolo[2',3':3,4]cyclopenta-[1,2-b]-pyridin (*Vergléichsbeispiel 1*) in 60 ml Acetonitril werden mit 5.42 ml 35 %iger Formaldehydlösung und anschließend portionsweie mit 1.04 g (16.5 mmol) Natriumcyanoborhydrid versetzt. Das Reaktionsgemisch wird 30 Minuten bei Raumtemperatur gerührt.
Nun wird mit 2n Salzsäure auf pH= 1 gestellt und zweimal mit je 30 ml Dichlormethan extrahiert. Die wäßrige Phase wird durch Zusatz von 2n Natronlauge auf pH> 11 gebracht und sechsmal mit je 30 ml Dichlormethan extrahiert. Man trocknet die organische Phase über Natriumsulfat/ Aktivkohle, filtriert und destilliert das Lösungsmittel ab.
***Ausbeute:*** 1.13 g gelbes Öl (90 % d. Th.)
***DC:*** Methanol: Ammoniak= 100: 2; R_{f} = 0.8
Methylenchlorid: Methanol= 10: 1; R_{f} = 0.5

Das Produkt wird mit alkoholischer Salzsäure in sein Dihydrochlorid überführt, unter Ethanol kristallisiert, abfiltriert und mit Aceton digeriert. Die erhaltenen farblosen Kristalle sind stark hygroskopisch.

### [α]_{D}²⁰: - 29.6 ± 1.0 ° (c = 0.44/ Methanol)

| ***Mikroelemtaranlyse:*** | RW16 | *C(%)* | *H(%)* | *N(%)* |
|---|---|---|---|---|
| *Summenformel:* | *berechnet:* | 48.83 | 6.93 | 10.35 |
| C₁₁H₁₆N₂Cl₂*1.30H₂O | *gefunden:* | 48.84 | 6.79 | 10.22 |

### ¹H- NMR (D₂O):

δ (ppm) = 8.78 (d,1H,Pcp-H6); 8.75 (d,1H,Pcp-H8); 7.96 (dd,1H,Pcp-H7); 5.28 (d,1H,Pcp-H8b); 3.85-3.50 (m,3H,Pcp-H2,4A); 3.19 (s,3H,CH₃); 3.50-3.00 (m,2H,Pcp-H4B,3a); 2.80- 2.50 (m,1H,Pcp-H3A); 2.05- 1.80 (m,1H,Pcp-H3B)

### ¹³C- NMR (D₂O):

δ (ppm) = 161.5 (s,Pcp-C4a); 146.8 (d,Pcp-C6); 145.5 (d,Pcp-C8); 137.2 (s,Pcp-C8a); 128.2 (d,Pcp-C7); 76.7 (d,Pcp-C8b); 59.4 (t,Pcp-C2); 43.1 (d,Pcp-C3a); 41.9 (q,CH₃); 38.4 (t,Pcp-C4); 32.9 (t,Pcp-C3)

### Vergleichsbeispiel 3

### (+)-[3aα, 8bα]-1, 2, 3, 3a, 4, 8b- Hexahydro-1-methyl-pyrrolo [2', 3': 3, 4] cyclopenta[1, 2-b]pyridin-Dihydrochlorid

aus 1.13 g (7.17 mMol) [3aα,8bα]-1,2,3,3a,4,8b-Hexahydropyrrolo[2',3':3,4]cyclopenta-[1,2-b]pyridin (*Vergleichsbeispiel 2*), 5.42 ml 35%iger Formaldehydlösung und 1.04 g (16.5 mMol) Natriumcyanoborhydrid analog zu Beispiel 3 (86% d.Th., farblose Kristalle)

### [α]_{D}²⁰: + 27.5 ± 0.5 ° (c = 0.42/ Methanol)

| ***Mikroelemtaranlyse:*** | RW8 | *C(%)* | *H(%)* | *N(%)* |
|---|---|---|---|---|
| *Summenformel:* | *berechnet:* | 48.96 | 6.92 | 10.38 |
| C₁₁H₁₆N₂Cl₂*1.26H₂O | *gefunden:* | 48.92 | 6.64 | 10.48 |

### ¹H- NMR (D₂O):

δ (ppm) = 8.78 (d,1H,Pcp-H6); 8.75 (d,1H,Pcp-H8); 7.96 (dd,1H,Pcp-H7); 5.28 (d,1H,Pcp-H8b); 3.85-3.50 (m,3H,Pcp-H2,4A); 3.19 (s,3H,CH₃); 3.50-3.00 (m,2H,Pcp-H4B,3a); 2.80- 2.50 (m,1H,Pcp-H3A); 2.05- 1.80 (m,1H,Pcp-H3B)

### ¹³C- NMR (D₂O):

δ (ppm) = 161.5 (s,Pcp-C4a); 146.8 (d,Pcp-C6); 145.5 (d,Pcp-C8); 137.2 (s,Pcp-C8a); 128.2 (d,Pcp-C7); 76.7 (d,Pcp-C8b); 59.4 (t,Pcp-C2); 43.1 (d,Pcp-C3a); 41.9 (q,CH₃); 38.4 (t,Pcp-C4); 32.9 (t,Pcp-C3)

### Vergleichsbeispiel 4

### (+)-[3aS-(3aα,8bα)]-1,2,3,3a,4,8b-Hexahydropyrrolo[3'2':4,5]-cyclopenta[1,2-c]-pyridin-Dihydrochlorid

6.00 g (250 mMol) Natriumhydrid werden bei 0 °C mit 240 ml abs. Pentanol versetzt und 30 Minuten gerührt. 4.80 g (15.6 mMol) [1S-[1R*(R*),2(R*)]]-1,2,3,3a,4,8b-Hexahydro-N-(1-phenylethyl)pyrrolo[3'2':4,5]cyclopenta[1,2-c]pyridin-1-carboxamid werden in fester Form auf einmal zugegeben und das Reaktionsgemisch zwei Stunden zum Sieden erhitzt.
Das Lösungsmittel wird bei 60 °C / 0.1 mbar abgezogen und der Rückstand rasch über 500 g Kieselgel KG60 (Methanol : Ammoniak = 100 : 2) filtriert. Das Rohprodukt wird säulenchromatographisch gereinigt (250 g Kieselgel KG60; Methanol : Ammoniak = 100 : 2). Das erhaltene Produkt wird in 20 ml Dichlormethan aufgenommen, über Natriumsulfat/Aktivkohle getrocknet, filtriert und das Lösungsmittel abdestilliert.
***Ausbeute:*** 1.93 g beige Kristalle (77 % d. Th.)
***DC:*** Methanol: Ammoniak= 100: 2; R_{f} = 0.25

Das Produkt wird mit alkoholischer Salzsäure in sein Dihydrochlorid überführt, unter Ethanol kristallisiert, abfiltriert und mit Aceton digeriert. Die erhaltenen farblosen Kristalle sind stark hygroskopisch.

### [α]_{D}²⁰: + 37.9° (c = 0.12 / Methanol)

| ***Mikroelemtaranlyse:*** | HA42 | *C(%)* | *H(%)* | *N(%)* |
|---|---|---|---|---|
| *Summenformel:* | *berechnet:* | 50.50 | 6.15 | 11.78 |
| C₁₀H₁₄C₁₂N₂*0.26H₂O | *gefunden:* | 50.64 | 6.08 | 11.55 |

### ¹H- NMR (D₂O):

δ (ppm) = 8.97 (s, 1H, Pcp-H8); 8.72 (d, 1H, Pcp-H6); 8.03 (d, 1H, Pcp-H5); 5.58 (d, 1H, Pcp-H8b); 3.78 - 3.40 (m, 3H, Pcp-H3a,4A,B); 3.38 - 3.18 (m, 2H, Pcp-H2A,B); 2.54 - 2.30 (m, 1H, Pcp-H3A); 2.04 - 1.87 (m, 1H, Pcp-H3B)

### ¹³C- NMR (D₂O):

δ (ppm) = 168.8 (s, Pcp-C4a); 143.3 (d, Pcp-C8); 141.1 (d, Pcp-C6); 138.0 (s, Pcp-C8a); 126.1 (d, Pcp-C5); 67.0 (d, Pcp-C8b); 47.8 (t, Pcp-C2); 42.6 (d, Pcp-C3a); 40.2 (t, Pcp-C4); 32.6 (t, Pcp-C3)

### Beispiel 2

### (-)-[3aR-(3aα,8bα)]-1,2,3,3a,4,8b-Hexahydropyrrolo[3'2':4,5]-cyclopenta[1,2-c]-pyridin-Dihydrochlorid

aus 1.00 g (3.26 mMol) [1R-[1R*(R*),2(S*)]]-1,2,3,3a,4,8b-Hexahydro-N-(1-phenylethyl)pyrrolo[3'2':4,5]cyclopenta[1,2-c]pyridin-1-carboxamid analog zu Vergleichsbeispiel 4 (83% d.Th., farblose Kristalle)

### [α]_{D}²⁰: -36.8 ° (c= 0.11 / Methanol)

| ***Mikroelemtaranlyse:*** | HA43 | *C(%)* | *H(%)* | *N(%)* |
|---|---|---|---|---|
| *Summenformel:* | *berechnet:* | 50.58 | 6.15 | 11.80 |
| C₁₀H₁₄Cl₂N₂ * 0.25 H₂O | *gefunden:* | 50.64 | 6.04 | 11.55 |

### ¹H- NMR (D₂O):

δ (ppm) = 8.97 (s, 1H, Pcp-H8); 8.72 (d, 1H, Pcp-H6); 8.03 (d, 1H, Pcp-H5); 5.58 (d, 1H, Pcp-H8b); 3.78 - 3.40 (m, 3H, Pcp-H3a,4A,B); 3.38 - 3.18 (m, 2H, Pcp-H2A,B); 2.54 - 2.30 (m, 1H, Pcp-H3A); 2.04 - 1.87 (m, 1H, Pcp-H3B)

### ¹³C- NMR (D₂O):

δ (ppm) = 168.7 (s, Pcp-C4a); 143.3 (d, Pcp-C8); 141.1 (d, Pcp-C6); 137.9 (s, Pcp-C8a); 126.0 (d, Pcp-C5); 67.0 (d, Pcp-C8b); 47.8 (t, Pcp-C2); 42.6 (d, Pcp-C3a); 40.2 (t, Pcp-C4); 32.5 (t, Pcp-C3)

***Das Ausgangsmaterial kann wie folgt hergestellt werden:***

### 6-Cyanomethyl-5,6-dihydro-7-oxo-7H-2-pyrindin-6-carbonsäuremethylester

Zu einer Suspension von 4.51 g (188 mMol) Natriumhydrid in 500 ml abs. Dimethylformamid werden bei 0 °C 30.00 g (157 mMol) 5,6-Dihydro-7-oxo-7H-2-pyrindin-6-carbonsäuremethylester portionsweise zugegeben. Die dabei entstehende Suspension wird zwei Stunden bei Raumtemperatur gerührt, bei 0 °C 31.40 g (188 mMol) Jodacetonitril zugegeben und das Gemisch über Nacht bei Raumtemperatur gerührt.
Das Lösungsmittel wird bei 60 °C / 0.1 mbar abgezogen und der Rückstand zwischen 500 ml Wasser und insgesamt 4 1 Diethylether verteilt. Die vereinigten organischen Phasen werden über Natriumsulfat/Aktivkohle getrocknet, filtriert und das Extraktionsmittel abgezogen. Das Rohprodukt wird aus 250 ml Ethanol umkristallisiert.
***Ausbeute:*** 23.50 g orange Kristalle (65 % d. Th.).
***DC:*** EE; R_{f} = 0.35
***Fp.:*** 102-103 °C (Ethanol)

| ***Mikroelemtaranlyse:*** | HA32 | *C(%)* | *H(%)* | *N(%)* |
|---|---|---|---|---|
| *Summenformel: .* | *berechnet:* | 62.61 | 4.48 | 12.17 |
| C₁₂H₁₀N₂O₃ | *gefunden:* | 62.39 | 4.33 | 12.14 |

### ¹H- NMR (CDCl₃):

δ (ppm) = 9.08 (s, 1H, Pn-H1); 8.82 (d, 1H, Pn-H3); 7.53 (dd, 1H, Pn-H4); 3.72 (s, 3H, OCH₃); 3.83 3.36 (AB, 1H, Pn-H5A); 3.21 2.29 (AB, 1H, CH_{A}H_{B}CN)

### ¹³C- NMR (CDCl₃):

δ (ppm) = 197.6 (s, C=O); 168.3 (s, COOCH₃); 160.1 (s, Pn-C4a); 154.9 (d, Pn-C1); 147.7 (d, Pn-C3); 129.8 (s, Pn-C7a); 121.7 (d, Pn-C4); 116.1 (s, CN); 56.6 (s, Pn-C6); 53.6 (q, OCH₃); 36.7 (t, Pn-C5); 22.1 (t, CH₂CN)

### 5,6-Dihydro-7-oxo-7H-2-pyrindin-6-essigsäurenitril

11.6 g (50.4 mMol) 6-Cyanomethyl-5,6-dihydro-7-oxo-7H-2-pyrindin-6-carbonsäuremethylester werden in 180 ml 2n Salzsäure 4 Stunden zum Sieden erhitzt, anschließend mit festem Natriumhydrogencarbonat auf pH = 9 gestellt und siebenmal mit je 100 ml Essigsäureethylester extrahiert. Die vereinigten organischen Phasen werden mit 100 ml Wasser gewaschen, über Natriumsulfat/Aktivkohle getrocknet, filtriert und das Lösungsmittel abgezogen. Das Produkt wird mehrmals mit Diethylether digeriert.
***Ausbeute:*** 7.20 g dunkelgrüne Kristalle (83 % d. Th.)
***DC:*** EE; R_{f} = 0.2
***Fp.:*** 94-95 °C (Diethylether)

| ***Mikroelemtaranlyse:*** | HA33 | *C(%)* | *H(%)* | *N(%)* |
|---|---|---|---|---|
| *Summenformel:* | *berechnet:* | 69.25 | 4.73 | 16.15 |
| C₁₀H₈N₂O * 0.07 H₂O | *gefunden:* | 69.26 | 4.86 | 16.00 |

### ¹H- NMR (CDCl₃):

δ (ppm) = 9.01 (s, 1H, Pn-H1); 8.75 (d, 1H, Pn-H3); 7.49 (dd, 1H, Pn-H4); 3.72 - 3.40 (m, 1H, Pn-H6); 3.14 - 2.89 (m, 3H, Pn-H5A,B, CH_{A}H_{B}CN); 2.77 - 2.60 (m, 1H, CH_{A}H_{B}CN)

### ¹³C- NMR (CDCl₃):

δ (ppm) = 202.2 (s, C=O); 160.0 (s, Pn-C4a); 153.9 (d, Pn-C1); 146.2 (d, Pn-C3); 130.9 (s, Pn-C7a); 121.6 (d, Pn-C4); 117.0 (s, CN); 42.3 (d, Pn-C6); 31.3 (t, Pn-C5); 17.7 (t, CH₂CN)

### (±)-[3aα,8bα]-1,2,3,3a,4,8b-Hexahydropyrrolo[3'2':4,5]cyclopenta[1,2-c]-pyridin

3.45 g (20.0 mMol) 5,6-Dihydro-7-oxo-7H-2-pyrindin-6-essigsäurenitril werden in 110 ml abs. Methanol gelöst, mit Aktivkohle gerührt, filtriert, mit 12 g Raney-Cobalt als Katalysator versetzt und in einer Parr-Apparatur bei 50 °C und 90 psi bis zum Ende der theoretischen Wasserstoffaufnahme hydriert.
Raney-Cobalt wird über Hyflo abfiltriert, das Lösungsmittel des Filtrats abdestilliert und der Rückstand säulenchromatographisch gereinigt (135 g Kieselgel KG60; Methanol : Ammoniak = 100 : 2).
***Ausbeute:*** 2.00 g braune Kristalle (62 % d. Th.)
***DC*:** Methanol: Ammoniak= 100: 2; R_{f}= 0.25

| ***Mikroelemtaranlyse:*** | HA34A | *C(%)* | *H(%)* | *N(%)* |
|---|---|---|---|---|
| *Summenformel:* | *berechnet:* | 74.97 | 7.55 | 17.48 |
| C₁₀H₁₂N₂ | *gefunden:* | 74.71 | 7.57 | 17.39 |

### ¹H- NMR (CDCl₃):

δ (ppm) = 8.56 (s, 1H, Pcp-H8); 8.38 (d, 1H, Pcp-H6); 7.09 (d, 1H, Pcp-H5); 4.85 (d, 1H, Pcp-H8b); 3.28 - 3.13 (m, 1H, Pcp-H3a); 3.13 - 2.90 (m, 2H, Pcp-H4A,B); 2.80 - 2.62 (m, 2H, Pcp-H2A,B); 2.34 (s_{breit}, 1H, NH); 2.10 - 1.95 (m, 1 H, Pcp-H3A); 1.63 - 1.47 (m, 1 H, Pcp-H3B)

### ¹³C- NMR (CDCl₃):

δ (ppm) = 152.6 (s, Pcp-C4a); 148.3 (d, Pcp-C8); 147.2 (d, Pcp-C6); 140.6 (s, Pcp-C8a); 119.9 (d, Pcp-C5); 67.0 (d, Pcp-C8b); 46.8 (t, Pcp-C2); 41.4 (d, Pcp-C3a); 38.5 (t, Pcp-C4); 35.6 (t, Pcp-C3)

### [1S-[1R*(R*),2(R*)]]-1,2,3,3a,4,8b-Hexahydro-N-(1-phenylethyl)pyrrolo-[3'2':4,5]cyclopenta[1,2-c]pyridin-1-carboxamid

10.15 g (63.4 mMol) (±)-[3aα,8bα]-1,2,3,3a,4,8b-Hexahydropyrrolo[3'2':4,5]cyclopenta-[1,2-c]-pyridin in 230 ml abs. Aceton werden mit 9.32 g (63.4 mMol) (S)-(-)-α-Methyl-benzolmethanisocyanat in 20 ml abs. Aceton versetzt, 30 Minuten bei Raumtemperatur gerührt und anschließend das Lösungsmittel abgezogen.
Das Rohprodukt wird in 500 ml Essigsäureethylester in der Siedehitze gelöst, über Aktivkohle 5 Minuten gerührt und filtriert. Das Filtrat wird mit Impfkristallen versetzt, und man läßt 3 Stunden bei -20 °C auskristallisieren. Die ausgefallenen Kristalle werden abfiltriert und zweimal mit je 5 ml eiskaltem Essigsäureethylester digeriert.
***Ausbeute:*** 5.00 g beige Kristalle (52 % d. Th.)
***DC:*** EE : MeOH = 8 : 1; 0.3
***Fp.:*** 163 - 164 °C (Essigsäureethylester)
***[*α*]_{D}²⁰:*** -218.3 ° (c = 0.12 / Dichlormethan)

| ***Mikroelemtaranlyse:*** | HA35 | *C(%)* | *H(%)* | *N(%)* |
|---|---|---|---|---|
| *Summenformel:* | *berechnet:* | 73.38 | 6.94 | 13.51 |
| C₁₉H₂₁N₃O*0.2H₂O | *gefunden:* | 73.50 | 6.88 | 13.51 |

### ¹H- NMR (CDCl₃):

δ (ppm) = 8.93 (s, 1H, Pcp-H8); 8.44 (d, 1H, Pcp-H6); 7.43 - 7.20 (m, 5H, Ph-H2,3,4,5,6); 7.13 (d, 1H, Pcp-H5); 5.46 (d, 1H, Pcp-H8b); 5.10 (dq, 1H, CH); 4.55 (d, 1H, NH, ³J_{H,CH} = 7.7Hz); 3.42 - 3.26 (m, 2H, Pcp-H2A,B); 3.18 - 2.96 (m, 2H, Pcp-H3a,4A); 2.75 (d, 1H, Pcp-H4B); 2.31 - 2.11 (m, 1H, Pcp-H3A); 1.75 - 1.57 (m, 1H, Pcp-H3B); 1.40 (d, 3H, CH₃)

### ¹³C- NMR (CDCl₃):

δ (ppm) = 156.1 (s, C=O); 150.4 (s, Pcp-C4a); 148.8 (d, Pcp-C8); 148.4 (d, Pcp-C6); 144.3 (s, Ph-C1); 140.1 (s, Pcp-C8a); 128.5 (d, 2C, Ph-C3,5); 127.0 (d, Ph-C4); 126.0 (d, 2C, Ph-C2,6); 120.2 (d, Pcp-C5); 65.0 (d, Pcp-C8b); 49.8 (d, CH); 45.8 (t, Pcp-C2); 40.9 (d, Pcp-C3a); 35.8 (t, Pcp-C4); 31.1 (t, Pcp-C3); 22.5 (q, CH₃)

### [1R-[1R*(R*),2(S*)]]-1,2,3,3a,4,8b-Hexahydro-N-(1-phenylethyl)-pyrrolo-[3'2':4,5]cyclopenta[1,2-c]pyridin-1-carboxamid

Die Mutterlauge des oberen Produktes engt man auf 300 ml ein, löst ggf. in der Siedehitze, versetztmit Impfkristallen und läßt 5 Stunden bei -20 °C auskristallisieren. Die ausgefallenen Kristalle werden abfiltriert und zweimal mit je 2 ml eiskaltem Essigsäureethylester digeriert.
***Ausbeute:*** 1.20 g farblose Kristalle (12 % d. Th.)
***DC:*** EE : MeOH = 8 : 1; 0.3
***Fp.:*** 154 - 155 °C (Essigsäureethylester)
***[*α*]_{D}²⁰:*** +185.6 ° (c = 0.13 / Dichlormethan)

| ***Mikroelemtaranlyse:*** | HA36 | *C(%)* | *H(%)* | *N(%)* |
|---|---|---|---|---|
| *Summenformel:* | *berechnet:* | 72.96 | 6.96 | 13.43 |
| C₁₉H₂₁N₃O*0.3H₂O | *gefunden:* | 73.05 | 6.88 | 13.34 |

### ¹H- NMR (CDCl₃):

δ (ppm) = 8.88 (s, 1H, Pcp-H8); 8.44 (d, 1H, Pcp-H6); 7.42 - 7.20 (m, 5H, Ph-H2,3,4,5,6); 7.13 (d, 1H, Pcp-H5); 5.40 (d, 1H, Pcp-H8b, ³J_{H,H3a} = 7.4Hz); 5.12 (dq, 1H, CH); 4.55 (d, 1H, NH); 3.45 - 3.25 (m, 2H, Pcp-H2A,B); 3.19 - 3.00 (m, 2H, Pcp-H3a,4A); 2.75 (d, 1H, Pcp-H4B); 2.31 - 2.15 (m, 1H, Pcp-H3A); 1.70 - 1.59 (m, 1H, Pcp-H3B); 1.57 (d, 3H, CH₃)

### ¹³C- NMR (CDCl₃):

δ (ppm) = 156.3 (s, C=O); 150.5 (s, Pcp-C4a); 148.9 (d, Pcp-C8); 148.6 (d, Pcp-C6); 144.2 (s, Ph-C1); 140.2 (s, Pcp-C8a); 128.6 (d, 2C, Ph-C3,5); 127.2 (d, Ph-C4); 126.1 (d, 2C, Ph-C2,6); 120.5 (d, Pcp-C5); 65.1 (d, Pcp-C8b); 49.8 (d, CH); 46.0 (t, Pcp-C2); 41.1 (d, Pcp-C3a); 35.9 (t, Pcp-C4); 31.3 (t, Pcp-C3); 22.5 (q, CH₃)

### Vergleichsbeispiel 5

### (+)-[3aS-(3aα,8bα)]-1,2,3,3a,4,8b-Hexahydro-1-methylpyrrolo-[3'2':4,5]cyclopenta[1,2-c]pyridin-Dihydrochlorid

650 mg (4.06 mMol) [3aS-(3aα,8bα)]-1,2,3,3a,4,8b-Hexahydropyrrolo[3'2':4,5]-cyclopenta[1,2-c]pyridin in 30 ml abs. Acetonitril werden mit 3.1 ml 35 %iger Formaldehydlösung und anschließend portionsweise mit 586 mg (9.33 mMol) Natriumcyanoborhydrid versetzt. Das Reaktionsgemisch wird 30 Minuten bei Raumtemperatur gerührt.
Nun wird mit 2n Salzsäure auf pH = 1 gestellt und zweimal mit je 30 ml Dichlormethan extrahiert. Die wäßrige Phase wird durch Zusatz von 2n Natronlauge auf pH = 10 gebracht und fünfmal mit je 50 ml Dichlormethan extrahiert. Man trocknet die organische Phase über Natriumsulfat/Aktivkohle, filtriert und destilliert das Lösungsmittel ab.
***Ausbeute:*** 650 mg gelbes Öl (92 % d. Th.)
***DC:*** Methylenchlorid: Methanol= 8: 1; R_{f} = 0.25

Das Produkt wird mit alkoholischer Salzsäure in sein Dihydrochlorid überführt, unter Ethanol kristallisiert, abfiltriert und mit Aceton digeriert. Die erhaltenen farblosen Kristalle sind stark hygroskopisch.

### [α]_{D}²⁰: +13.2 ° (c = 0.11 / Methanol)

| ***Mikroelemtaranlyse:*** | HA46 | *C(%)* | *H(%)* | *N(%)* |
|---|---|---|---|---|
| *Summenformel:* | *berechnet:* | 52.20 | 6.63 | 11.07 |
| C₁₁H₁₆Cl₂N₂*0.33H₂O | *gefunden:* | 52.25 | 6.71 | 10.88 |

### ¹H- NMR (D₂O):

δ (ppm) = 9.08 (s, 1H, Pcp-H8); 8.76 (d, 1H, Pcp-H6); 8.05 (d, 1H, Pcp-H5); 5.50 - 5.26 (m, 1H, Pcp-H8b); 3.86 - 3.20 (m, 5H, Pcp-H2A,B,3a,4A,B); 3.15 (s, 3H, CH₃); 2.76 - 2.48 (m, 1H, Pcp-H3A); 2.10 - 1.81 (m, 1H, Pcp-H3B)

### ¹³C- NMR (D₂O):

δ (ppm) = 168.8 (s, Pcp-C4a); 143.9 (d, Pcp-C8); 140.8 (d, Pcp-C6); 136.5 (s, Pcp-C8a); 126.3 (d, Pcp-C5); 76.5 (d, Pcp-C8b); 58.6 (t, Pcp-C2); 42.8 (q, CH₃); 42.0 (d, Pcp-C3a); 40.6 (t, Pcp-C4); 31.9 (t, Pcp-C3)

### Beispiel 3

### (-)-[3aR-(3aα,8bα)]-1,2,3,3a,4,8b-Hexahydro-1-methylpyrrolo-[3'2':4,5]cyclopenta[1,2-c]pyridin-Dihydrochlorid

aus 580 mg (3.62 mMol) [3aR-(3aα,8bα)]-1,2,3,3a,4,8b-Hexahydropyrrolo[3'2':4,5]-cyclopenta[1,2-c]pyridin, 2.74 ml 35%iger Formaldehydlösung und 523 mg (8.33 mMol) Natriumcyanoborhydrid analog zu Beispiel 7 (90 % d. Th., farblose Kristalle)

### [a]_{D}²⁰: -14.0 ° (c = 0.14 / Methanol)

| ***Mikroelemtaranlyse:*** | HA47 | *C(%)* | *H(%)* | *N(%)* |
|---|---|---|---|---|
| *Summenformel:* | *berechnet:* | 52.42 | 6.61 | 11.11 |
| C₁₁H₁₆Cl₂N₂*0.27H₂O | *gefunden:* | 52.47 | 6.80 | 10.94 |

### ¹H- NMR (D₂O):

δ (ppm) = 9.08 (s, 1H, Pcp-H8); 8.76 (d, 1H, Pcp-H6); 8.05 (d, 1H, Pcp-H5); 5.50 - 5.26 (m, 1H, Pcp-H8b); 3.86 - 3.20 (m, 5H, Pcp-H2A,B,3a,4A,B); 3.15 (s, 3H, CH₃); 2.76 - 2.48 (m, 1H, Pcp-H3A); 2.10 - 1.81 (m, 1H, Pcp-H3B)

### ¹³C- NMR (D₂O):

δ (ppm) = 168.8 (s, Pcp-C4a); 143.8 (d, Pcp-C8); 140.8 (d, Pcp-C6); 136.7 (s, Pcp-C8a); 126.3 (d, Pcp-C5); 76.5 (d, Pcp-C8b); 58.7 (t, Pcp-C2); 42.8 (q, CH₃); 42.1 (d, Pcp-C3a); 40.6 (t, Pcp-C4); 31.9 (t, Pcp-C3)

### Beispiel 4

### Radioligand Assay

Die [³H] Cytisin-Bindung an den α4β2-Subtyp in Rattenhirnmembranen wurde mittels einer modifizierten Methode von Pabreza et. Al. (Pabreza, L.A., Dhawan, S., Kellar, K.J., Mol. Pharmacol. 1991, 39, 9-12) bestimmt:

Membran-angereicherte Fraktionen von Rattenhirn ohne Cerebellum (ABS Inc. Wilmington, DE) wurde langsam auf 4°C aufgetaut, gewaschen und wieder in 30 Teilen BSS-Tris-Buffer (120 mM NaCl, 5mM KCl, 2 mM CaCl₂, 2 mM MgCl₂ und 50 mM Tris-Cl, pH 7,4, 4°C) suspendiert. Verdünnungen der Testverbindung (10⁻⁵ bis 10⁻¹¹ M), die 100-200 µg Protein und 0,75 nM [³H] Cytisin (30 Ci/mmol; Perkin Elmer NEN, Boston, MA) enthalten, wurden in einem Endvolumen von 500 µl für 75 Minuten bei 4°C inkubiert (jeweils 2 Proben). Die nicht spezifische Bindung wurde mit 10 µM (-)-Nikotin bestimmt. Die Inkubierung wurde mittels Vakuumfiltration durch einen Whatman GF/C-Filter bestimmt, der vorher mit 0,5%igem Polyethylenimin angefeuchtet wurde. Gebundene Radioaktivität wurde auf Millipore Mulitscreen Platten FB mit einem Packard Cell Harvester gesammelt, und mit einem Packard Topcount Microplate Beta Counter bestimmt. IC₅₀-Werte wurden durch nicht-lineare Regression bestimmt und daraus die Kᵢ-Werte mittels der Cheng-Prusoff Gleichung, wobei Kᵢ = IC₅₀ / 1 + [Ligand] / K_{D}. Die mittleren Kᵢ-Werte wurden aus mindestens drei Einzelbestimmungen gewonnen.

| **Kᵢ-Werte [nM] der Enantiomerenpaare** | | | |
|---|---|---|---|
| Vergleichsbeispiel 1 | 2430 | Vergleichsbeispiel 2 | > 1 mM |
| Beispiel 1 | 199 | Vergleichsbeispiel 3 | > 1 mM |
| Vergleichsbeispiel 4 | 1110 | Beispiel 2 | 15,2 |
| Vergleichsbeispiel 5 | 1670 | Beispiel 3 | 27,0 |

## Patentansprüche

1. Neue therapeutisch wertvolle enantiomerenreine Verbindungen (-)-[3aα,8bα]-1,2,3,3a,4,8b-Hexahydro-1-methyl-pyrrolo[2',3':3,4]-cyclopenta[1,2-b]pyridin, (-)-[3aR-(3aα,8bα)]-1,2,3,3a,4,8b-Hexahydro-pyrrolo[3',2':4,5]-cyclopenta[1,2-c]pyridin oder (-)-[3aR-(3aα,8bα)]-1,2,3,3a,4,8b-Hexahydro-1-methyl-pyrrolo[3',2':4,5]-cyclopenta[1,2-c]pyridin, sowie deren pharmazeutisch verwendbaren Salze.

2. (-)-[3aα,8bα]-1,2,3,3a,4,8b-Hexahydro-1-methyl-pyrrolo[2',3':3,4]-cyclopenta[1,2-b]pyridin-Dihydrochlorid.

3. (-)-[3aR-(3aα,8bα)]-1,2,3,3a,4,8b-Hexahydro-pyrrolo[3',2':4,5]-cyclopenta[1,2-c]pyri din-Dihydrochlorid.

4. (-)-[3aR-(3aα,8bα)]-1,2,3,3a,4,8b-Hexahydro-1-methyl-pyrrolo[3',2':4,5]-cyclopenta[1,2-c]pyridin-Dihydrochlorid.

5. Ein Verfahren zur Herstellung von Verbindungen nach einem der Ansprüche 1 bis 4, welches **dadurch gekennzeichnet ist, dass** man eine Verbindung der allgemeinen Formel 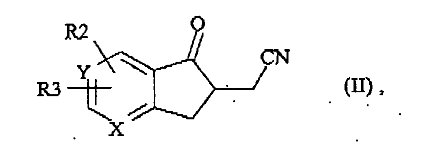 worin R2, R3 Wasserstoff sind und X und Y alternierend CH oder N bedeuten, reduktiv in die Verbindung der allgemeinen Formel (I) 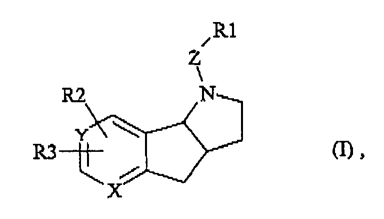 mit Z = Einfachbindung und R1 = Wasserstoff überführt, diese gegebenenfalls mit enantiomerenreinem 1-Phenylethylisocyanat zur Verbindung der allgemeinen Formel 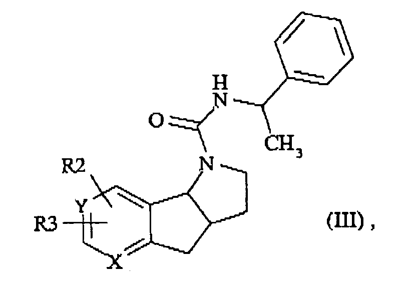 umsetzt, aus dem so erhaltenen Diastereomerengemisch das schwerer lösliche Diastereomere durch Kristallisation gewinnt, die so erhaltene diastereomerenreine Verbindung der allgemeinen Formel (III) unter geeigneten Bedingungen zur enantiomerenreinen Verbindung der allgemeinen Formel (I) mit Z = Einfachbindung und R1 = Wasserstoff spaltet, diese gegebenenfalls unter alkylierenden Bedingungen zu Verbindungen der allgemeinen Formel (I) mit Z = CH₂ umsetzt, und gegebenenfalls die Verbindung der allgemeinen Formel (I) in ihre pharmazeutisch verwendbaren Salze überführt.

6. Pharmazeutische Präparate, enthaltend Verbindungen nach einem der Ansprüche 1 bis 4, sowie deren Salze in Kombination mit üblichen galenischen Hilfs- und/oder Trägerstoffen.

7. Pharmazeutische Präparate nach Anspruch 6, in Kombination mit anderen therapeutisch wertvollen Verbindungen sowie Hilfs- und/oder Trägerstoffen.

8. Verbindungen nach Anspruch 1, zur Verwendung als Wirkstoffe für Arzneimittel zur Behandlung von Erkrankungen des zentralen Reizleitungssystemes wie z.B. altersbedingte Demenz oder Alzheimer-Krankheit.

9. Verbindungen nach Anspruch 1, zur Verwendung als Wirkstoffe für Arzneimittel zur Behandlung von Erkrankungen des zentralen Reizleitungssystemes wie z.B. Parkinson-Krankheit, Tourett's-Syndrom oder Dyskinesie.

10. Verbindungen nach Anspruch 1, zur Verwendung als Wirkstoffe für Arzneimittel zur Behandlung von Erkrankungen des zentralen Reizleitungssystemes wie z.B. Angstzustände, Depression, Panik, Psychosen, Bulemie oder Anorexie.

11. Verbindungen nach Anspruch 1, zur Verwendung als Wirkstoffe für Arzneimittel zur Behandlung von Erkrankungen des zentralen Reizleitungssystemes wie z.B. als Analgetika, Nozizieptiva, Neuroprotektiva, zur Verbesserung von Wahrnehmung und Aufmerksamkeit sowie in der Rauch-Ersatztherapie.

## Claims

1. Novel therapeutically valuable enantiomerically pure compounds (-)-[3aα,8bα]-1,2,3,3a,4,8b-hexahydro-1-methyl-pyrrolo[2',3':3,4]-cyclopenta[1,2-b]pyridine, (-)-[3aR-(3aα,8bα)]-1,2,3,3a,4,8b-Hexahydropyrrolo[3',2':4,5]-cyclopenta[1,2-c]pyridine or (-)-[3aR-(3aα,8bα)]-1,2,3,3a,4,8b-Hexahydro-1-methyl-pyrrolo[3',2':4,5]-cyclopenta[1,2-c]pyridine as well as the pharmaceutically usable salts thereof.

2. (-)-[3aα,8bα]-1,2,3,3a,4,8b-Hexahydro-1-methyl-pyrrolo-[2',3':3,4]-cyclopenta[1,2-b]pyridine dihydrochloride.

3. (-)-[3aR-(3aα,8bα)]-1,2,3,3a,4,8b-hexahydropyrrolo[3',2':4,5]-cyclopenta[1,2-c]pyridine dihydrochloride.

4. (-)-[3aR-(3aα,8bα)]-1,2,3,3a,4,8b-Hexahydro-1-methyl-pyrrolo[3',2':4,5]-cyclopenta[1,2-c]pyridine dihydrochloride.

5. A method for producing compounds according to any one of claims 1 to 4, which is **characterized in that** a compound of the general formula 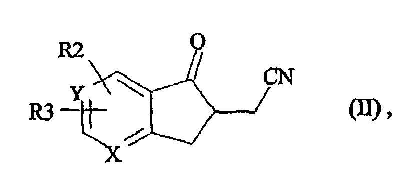 wherein R2, R3 are hydrogen, and X and Y alternately represent CH or N, is reductively converted into the compound of the general formula (I) 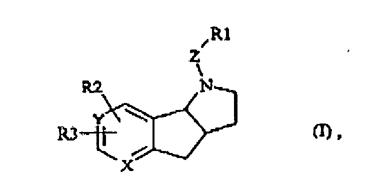 wherein Z = single bond and R1 = hydrogen, the latter compound optionally is reacted with enantiomerically pure 1-phenylethyl-isocyanate to give the compound of the general formula 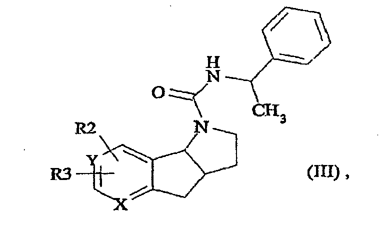 the less readily soluble diastereomer is recovered from the thus-obtained diastereomer mixture by crystallization, and the diastereomerically pure compound of the general formula (III) thus obtained is cleaved under suitable conditions to give the enantiomerically pure compound of the general formula (I), wherein Z = a single bond and R1 = hydrogen, the latter compound optionally is reacted under alkylating conditions to compounds of the general formula (I), wherein Z = CH₂, and the compound of the general formula (I) optionally is converted into its pharmaceutically usable salts.

6. Pharmaceutical preparations, containing compounds according to any one of claims 1 to 4, as well as their salts in combination with common galenic auxiliary and/or carrier substances.

7. Pharmaceutical preparations according to claim 6, in combination with other therapeutically valuable compounds as well as auxiliary and/or carrier substances.

8. Compounds according to claim 1 to be used as active substances for medicaments for the treatment of diseases of the central conduction system, such as, e.g., dementia caused by old age or Alzheimer's Disease.

9. Compounds according to claim 1 to be used as active substances for medicaments for the treatment of diseases of the central conduction system, such as, e.g., Parkinson Disease, Tourett's Syndrome or dyskinesia.

10. Compounds according to claim 1 to be used as active substances for medicaments for the treatment of diseases of the central conduction system, such as, e.g., anxiety, depression, panic, psychosis, bulimia or anorexia.

11. Compounds according to claim 1 to be used as active substances for medicaments for the treatment of diseases of the central conduction system, such as, e.g., as analgesics, nociceptive agents, neuroprotective agents, for the improvement of perception and attention as well as in smoke substitution therapy.

## Revendications

1. Nouveaux composés énantiomériquement purs thérapeutiquement intéressants (-)-[3aa,8ba]-1,2,3,3a,4,8b-hexahydro-1-méthyl-5pyrrolo[2',3':3,4]-cyclopenta[1,2-b]pyridine, (-)-[3aR-(3aα,8bα)]-1,2,3,3a,4,8b-hexahydro-pyrrolo[3',2':4,5]-cyclopenta[1,2-c]pyridine ou (-)-[3aR-(3aα,8bα)]-1,2,3,3a,4,8b-hexahydro-1-méthyl-pyrrolo[3',2':4,5]-cyclopenta[1,2-c]pyridine, ainsi que leurs sels pharmaceutiquement utilisables.

2. Dichlorhydrate de (-)-[3aα,8bα]-1,2,3,3a,4,8b-hexahydro-1-méthyl-pyrrolo[2',3':3,4]-cyclopenta[1,2-b]pyridine.

3. Dichlorhydrate de (-)-[3aR-(3aα,8bα)]-1,2,3,3a,4,8b-hexahydro-pyrrolo[3',2':4,5]-cyclopenta[1,2-c]pyridine.

4. Dichlorhydrate de (-)-[3aR-(3aα,8bα)]-1,2,3,3a,4,8b-hexahydro-1-méthyl-pyrrolo[3',2':4,5]-cyclopenta[1,2-c]pyridine.

5. Procédé de production de composés selon l'une des revendications 1 à 4 qui est **caractérisé en ce que** l'on convertit un composé de formule générale 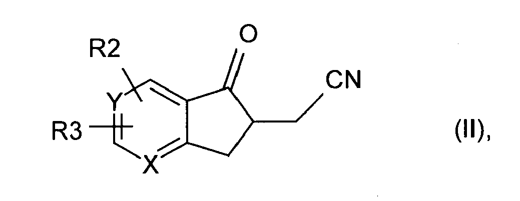 où R2, R3 sont l'hydrogène et X et Y représentent tour à tour CH ou N, par réduction en le composé de formule générale (I) 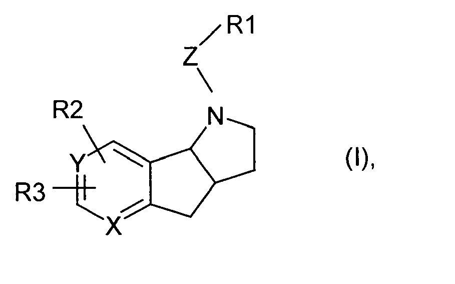 avec Z = simple liaison et R1 = hydrogène, on convertit celui-ci éventuellement avec l'isocyanate de 1-phényléthyle énantiomériquement pur en le composé de formule générale 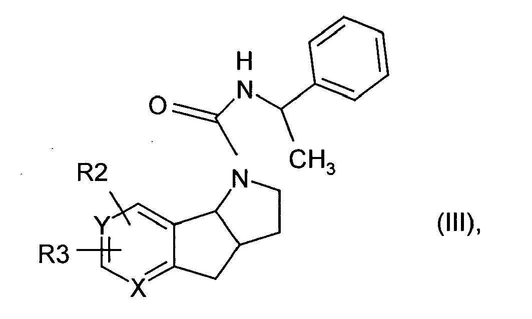 à partir du mélange de diastéréoisomères ainsi obtenu, on obtient par cristallisation le diastéréoisomère peu soluble, on clive le composé diastéréoisomériquement pur de formule générale (III) ainsi obtenu dans des conditions appropriées en le composé énantiomériquement pur de formule générale (I) avec Z = simple liaison et R1 = hydrogène, on convertit celui-ci éventuellement dans des conditions alkylantes en les composés de formule générale (I) avec Z = CH₂, et éventuellement on convertit le composé de formule générale (I) en ses sels pharmaceutiquement utilisables.

6. Préparation pharmaceutique contenant des composés selon l'une des revendications 1 à 4 ainsi que leurs sels en combinaison avec des adjuvants et/ou vecteurs galéniques courants.

7. Préparation pharmaceutique selon la revendication 6 en combinaison avec d'autres composés thérapeutiquement intéressants ainsi que des adjuvants et/ou vecteurs.

8. Composés selon la revendication 1 destinés à être utilisés comme principes actifs pour des médicaments pour le traitement des maladies du système nerveux central comme par exemple la démence due à l'âge ou la maladie d'Alzheimer.

9. Composés selon la revendication 1 destinés à être utilisés comme principes actifs pour des médicaments pour le traitement des maladies du système nerveux central comme par exemple la maladie de Parkinson, le syndrome de Tourette ou la dyskinésie.

10. Composés selon la revendication 1 destinés à être utilisés comme principes actifs pour des médicaments pour le traitement des maladies du système nerveux central comme par exemple les états de peur, la dépression, la panique, la psychose, la boulimie ou l'anorexie.

11. Composés selon la revendication 1 destinés à être utilisés comme principes actifs pour des médicaments pour le traitement des maladies du système nerveux central, par exemple comme analgésiques, nociceptifs, neuroprotecteurs, pour améliorer la perception et l'attention ainsi que dans la thérapie de substitution de la fumée.
